# EUROPEAN PATENT APPLICATION

(11) **EP 4 269 615 A1**
(43) Date of publication of application: **01.11.2023**
(21) Application number: 21909593.2
(22) Date of filing: 25.12.2021
(51) Int. Cl.: C12Q 1/6869

(54) **BIOCHIP FOR SPATIAL TRANSCRIPTOMIC ANALYSIS, MANUFACTURING METHOD THEREFOR AND APPLICATION THEREOF**

(30) Priority: 25.12.2020 CN 202011568787
(71) Applicant: Intelligent Healthcare Technology Limited, Hong Kong (HK)
(72) Inventor: ZHAO, Haifeng, Hong Kong (HK)
(74) Representative: Laine IP Oy
(86) International application number: PCT/CN2021/141391
(87) International publication number: WO 2022/135598

(57) **Abstract**

A method for manufacturing a chip for analyzing nucleic acid information of a biological sample. The chip is suitable for analyzing spatial transcriptomic information of a biological tissue sample. Also provided are the chip for analyzing the nucleic acid information of the biological sample and a method for using the chip to analyze the spatial transcriptomic information of the biological tissue sample. Nucleic acid expression information in cells of the tissue sample can be effectively obtained, and comprises spatial omics information.

## Description

This application claims priority to Chinese patent application No. 202011568787.3, filed on December 25, 2020, entitled "Method for preparing a biochip for spatial transcriptomics analysis", the entire contents of which are incorporated herein by reference.

### Technical Field

The present invention relates to the fields of biology and medical devices. Specifically, the present invention relates to a method of preparing a microarray for analyzing nucleic acid information of cells of a biological sample, said microarray being suitable for analyzing spatial transcriptomics information of a biological tissue sample.

### Background

The analysis of cellular organization and expression patterns of biological tissues is a milestone in biomedical research and diagnostics. Using a variety of staining techniques, cellular histology studies identified the basic structural mechanisms in healthy organs and common pathological changes more than a century ago. Developments in the field have led to the possibility of studying protein distribution by immunohistochemistry and gene expression by in situ hybridization.

New studies hope to characterize the transcriptome and/or genomic variation in tissues while preserving spatial information about the tissues.

Recent developments in gene expression analysis have opened up the possibility of using microarrays or RNA sequencing to assess the complete transcriptome of a tissue. However, typical transcriptome analyses are performed with mRNA extracted from whole tissues (or even intact organisms), but the collection of smaller tissue regions or individual cells for transcriptome analyses is generally very laborious, expensive, and of low accuracy.

### Summary

The present invention provides a method of preparing a microchip for analyzing nucleic acid information of cells of a biological sample, in particular suitable for analyzing spatial transcriptomics information of a biological tissue sample. Specifically, the present invention provides a method for preparing a biochip having an array, comprising the steps of:
A. fixing a first set of barcode nucleic acids on the surface of a chip through a plurality of microfluidic channels set in parallel to form a plurality of first barcode strips in a first direction, wherein said first set of barcode nucleic acids comprise a plurality of first barcode nucleic acids having different barcode sequences, each said first barcode strip contains one kind of first barcode nucleic acid, and each said kind of first barcode nucleic acid fixed in each first barcode strip has a different barcode sequence;
B. applying a second set of barcode nucleic acids in a second direction to said plurality of first barcode strips in the first direction on the chip surface through a plurality of microfluidic channels set in parallel to form a plurality of second barcode strips, said second set of barcode nucleic acids comprise a plurality of second barcode nucleic acids having different barcode sequences, each said second barcode strip contains one kind of second barcode nucleic acid, and each said kind of second barcode nucleic acid contained in each second barcode strip has a different barcode sequence;
C. under conditions that cause the first barcode nucleic acid and the second barcode nucleic acid to undergo a joining reaction, joining the second barcode nucleic acid to the first barcode nucleic acid on the surface of the chip where said plurality of first barcode strips intersect said plurality of second barcode strips to generate probes, said probes forming arrays of dots, each dot containing a probe with a sequence different from each other.

In one of the aspects of the present invention, in aforementioned method, said first set of barcode nucleic acids or second set of barcode nucleic acids are delivered and fixed to the chip surface using a microfluidic device having a plurality of microfluidic channels set in parallel, wherein the side of said microfluidic channel in contact with the chip surface is set to allow passage of solution or nucleic acids in solution.

In one of the aspects of the present invention, in aforementioned method, one of the first set of barcode nucleic acids or the second set of barcode nucleic acids containing different barcode sequences is added into each microfluidic channel of said microfluidic device.

In one of the aspects of the present invention, in aforementioned method, said first barcode nucleic acid of said first set of barcode nucleic acids comprises a first barcode fragment, and preferably comprises a primer fragment at the 5' end for use in an amplification reaction.

In one of the aspects of the present invention, in aforementioned method, said first barcode nucleic acid of said first set of barcode nucleic acids has a moiety at the 5' end for attachment to the surface of the chip.

In one of the aspects of the present invention, in aforementioned method, said second barcode nucleic acid in said second set of barcode nucleic acids comprises a capture fragment at the 3' end for recognizing and binding a target nucleic acid in the biological sample (e.g., a fragment for recognizing and binding mRNA or cDNA, e.g., a poly-T sequence) and a second barcode fragment.

In one of the aspects of the present invention, in aforementioned method, said second barcoded nucleic acid in said second set of barcoded nucleic acids further has a unique molecular identifier (UMI).

In one of the aspects of the present invention, in aforementioned method, said first barcode nucleic acid has a first linking fragment at the 3' end for linking to the second barcode nucleic acid via a single-stranded linking nucleic acid, and said second barcode nucleic acid has a second linking fragment at the 5' end for linking to the first barcode nucleic acid via said single-stranded linking nucleic acid, said first linking fragment and second linking fragment are each complementary to a sequence at each end of said single-stranded linking nucleic acid.

In one of the aspects of the present invention, in aforementioned method, the probe formed in step C comprises a capture fragment at the 3' end for identifying and binding a target nucleic acid in the biological sample, and a first barcode fragment and a second barcode fragment. Preferably said probe further has a primer fragment at the 5' end for use in an amplification reaction.

In one of the aspects of the present invention, in aforementioned method, the sequence of the barcode fragment of each of said first barcode nucleic acids of said first set of barcode nucleic acids is designated, and/or the sequence of the barcode fragment of each of said second barcode nucleic acids of said second set of barcode nucleic acids is designated.

In one of the aspects of the present invention, in aforementioned method, the sequence of the barcode fragment of each of said first barcode nucleic acids of said first set of barcode nucleic acids and the sequence of the barcode fragment of each of said second barcode nucleic acids of said second set of barcode nucleic acids is designated.

In one of the aspects of the present invention, in aforementioned method, the concentration of nucleic acid in the flow channel in step A is about 0.1-100 µM, for example, about 1-20 µM.

In one of the aspects of the present invention, in aforementioned method, the concentration of nucleic acid in the flow channel in step B is about 0.1-100 µM, for example, about 1-20 µM.

In one of the aspects of the present invention, in step A of aforementioned method, said nucleic acid of said first set of barcoded nucleic acids is fixed on the chip surface. Preferably, said nucleic acid of said first set of barcoded nucleic acids is fixed on the chip surface by chemical bonding means. The chemical bonding means is any one of chemical group reactions, for example, amino-aldehyde group reaction and the like, and covalent crosslinking. The surface of the chip may be encapsulated by a surface chemical reaction with an amino group, an aldehyde group, an epoxy group, an isothiocyanate group, a sulfhydryl group, a silane, and other reactive groups, etc.; and the end of the nucleic acid of said first set of barcoded nucleic acids that is connected to the surface of the chip (usually the 5' end) has a group that forms a chemical bond with the encapsulated reactive group.

In one of the aspects of the present invention, the width of each channel of said microfluidic channels set in parallel in step A and step B is about 2-200 µm, preferably about 5-50 µm, most preferably about 5-25 µm, for example about 5 µm, 10 µm or 50 µm.

In one of the aspects of the present invention, the spacing between each adjacent microfluidic channel of the microfluidic channels set in parallel is about 5-400 µm, preferably about 10-100 µm, most preferably about 10-50 µm, such as about 20 µm, 50 µm or 100 µm.

The microarrays obtained by the preparation of the present invention can be used for the analysis of tissue samples, in particular tissue thin sections, for the analysis of molecules contained in cells, including the analysis of nucleic acids and proteins, for example by PCR, mass spectrometry, next-generation sequencing, or ELISA, to obtain expression and spatial information thereof.

In one of the aspects of the present invention, said biological sample is a tissue sample from a subject, for example a surgically excised tissue sample, preferably a thin section of tissue obtained by microtome processing. In one aspect of the present invention, said tissue sample is fixed and embedded (e.g. embedded in paraffin), and attached to a support such as a slide.

In one aspect of the present invention, said tissue thin section may be subjected to morphological analysis and/or histological analysis, which is performed by H&E staining, IHC staining, ISH staining, or FISH staining.

In one aspect of the present invention, the analysis of the one or more biomolecules is performed by PCR, mass spectrometry, next generation sequencing, or ELISA.

In one aspect of the present invention, the subject is selected from an animal, a farm animal, a pet, a human subject.

In one aspect of the present invention, the analyte further comprises one or more of a non-human cell, a human cell, an unnatural protein, a nucleic acid, or a small molecule, a dye, a virus, a bacterium, a parasite, a protozoan, or a chemical substance. Small molecules include semi-antigens, peptide tags, protein tags, fluorescent tags, nucleic acid tags, and combinations thereof.

In one aspect of the present invention, said microarray may be used to analyze quantitative and/or qualitative data for a marker in a sample. The marker comprises DNA, proteins, RNA, lipids, organelles, metabolites, or cells. The protein may comprise a modification, said modification being selected from the group consisting of: acetylation, ADP-ribosylation, acylation, ADP-ribosylation, amidation, covalent attachment to flavin, covalent attachment to heme, covalent attachment to nucleotides or nucleotide derivatives, covalent attachment to lipids or lipid derivatives, covalent attachment to phosphatidylinositol, crosslinking, cyclization, disulfide bond formation, demethylation, formation of a covalent crosslinks, cystine formation, pyroglutamic acid formation, formylation, γ-carboxylation, glycosylation, GPI anchor formation, hydroxylation, iodination, methylation, myristoylation, oxidation, protein hydrolysis processing, phosphorylation, isopentenylation, racemization, selenylation, sulfation, argininylation, and ubiquitination. The markers include genomic polymorphisms, pharmacogenomic single nucleotide polymorphisms (SNPs), genomic SNPs, somatic polymorphisms, and differential expression of proteins, lipids, and/or organelles. The markers include individual nucleotide positions; intragenic regions or intergenic regions; exons or introns, or fragments thereof; coding or non-coding regions; promoters, enhancers, 5 ' untranslated regions (5 'UTRs), or 3 'untranslated regions (3 'UTRs), or fragments thereof; cDNAs or fragments thereof; SNPs; somatic mutations, germline mutations, or both; point mutations or single mutations; deletion mutation; in-frame deletion, in-gene deletion, whole gene deletion; insertion mutation; in-gene insertion; inversion mutation; intrachromosomal inversion; linked mutation; linked insertion mutation; inverted repeat mutation; tandem duplication; intrachromosomal tandem duplication; translocation; chromosomal translocation, non-mutual translocation; rearrangement; genome rearrangement; rearrangement of one or more introns, or fragments thereof; rearrangement of an intron; a 5 '- or 3 ' -UTR, or a combination thereof. Wherein the marker comprises an altered nucleotide sequence coding for an altered amino acid sequence, a chromosomal translocation, an intrachromosomal inversion, a copy number change, an expression level change, a protein level change, a protein activity change, or a methylation status change in a cancerous tissue or a cancerous cell as compared to a normal healthy tissue or cell. Wherein the marker may be measured by single cell sequencing, single nucleus sequencing, flow cytometry, immunohistochemical staining, hematoxylin and eosin staining, whole genome sequencing, high throughput sequencing, mass spectrometry, DNA microarrays, or combinations thereof.

The microchip of the present invention may be used to analyze a tissue sample. The tissue sample comprises a sample selected from the group consisting of: one or more premalignant or malignant cells, cells from solid tumors, soft tissue tumors or metastases, tissue or cells from surgical margins, histologically normal tissue, one or more circulating tumor cells (CTCs), normal adjacent tissue (NAT), blood samples from the same subject suffering from, or at risk of, developing a tumor, or FFPE samples.

### Brief description of the drawings

In order to more clearly illustrate the technical solutions in the embodiments or prior art of the present invention, the following will be a brief introduction to the accompanying drawings that need to be used in the description of the embodiments or prior art. It is obvious that the accompanying drawings in the following description are some embodiments of the present invention, and for the person of ordinary skill in the field, other accompanying drawings can be obtained according to these drawings without giving creative labor.
FIG. 1 shows a schematic diagram of the steps of a method for preparing a chip provided by the present invention.
FIG. 2 shows an exemplary embodiment of the method of the present invention using an apparatus having a plurality of microfluidic channels disposed in parallel.
FIG. 3 shows a schematic diagram of the steps of a method for preparing a biochip provided by the present invention.
FIG. 4 shows a schematic flow diagram of an exemplary embodiment of a method of preparing a biochip provided by the present invention.
FIG. 5 shows a schematic flow diagram of yet another exemplary embodiment of a method of preparing a biochip provided by the present invention.
FIG. 6 shows an observation of the preparation process of the prepared biochip provided by the present invention on its barcode nucleic acid ligation reaction.
FIG. 7 shows an image of a sample tissue after HE staining of tissue sections in a spatial transcriptomics study of the sample tissue using the biochip provided by the present invention.

### Examples

In order to make the objects, technical solutions and advantages of the embodiments of the present invention clearer, the technical solutions in the embodiments of the present invention will be described clearly and completely in the following in conjunction with the accompanying drawings in the embodiments of the present invention, and it is clear that the described embodiments are a part of the embodiments of the present invention and not all of the embodiments. Based on the embodiments in the present invention, all other embodiments obtained by a person of ordinary skill in the art without making creative labor fall within the zone of protection of the present invention.

### Example 1

The present invention provides a method for preparing a biochip suitable for analyzing nucleic acid information of cells of a biological sample. More specifically, the present invention provides a method for preparing a biochip having an array. In one aspect of the present invention, the chip provided by the present invention is adapted to analyze spatial transcriptomics information of a biological tissue sample.

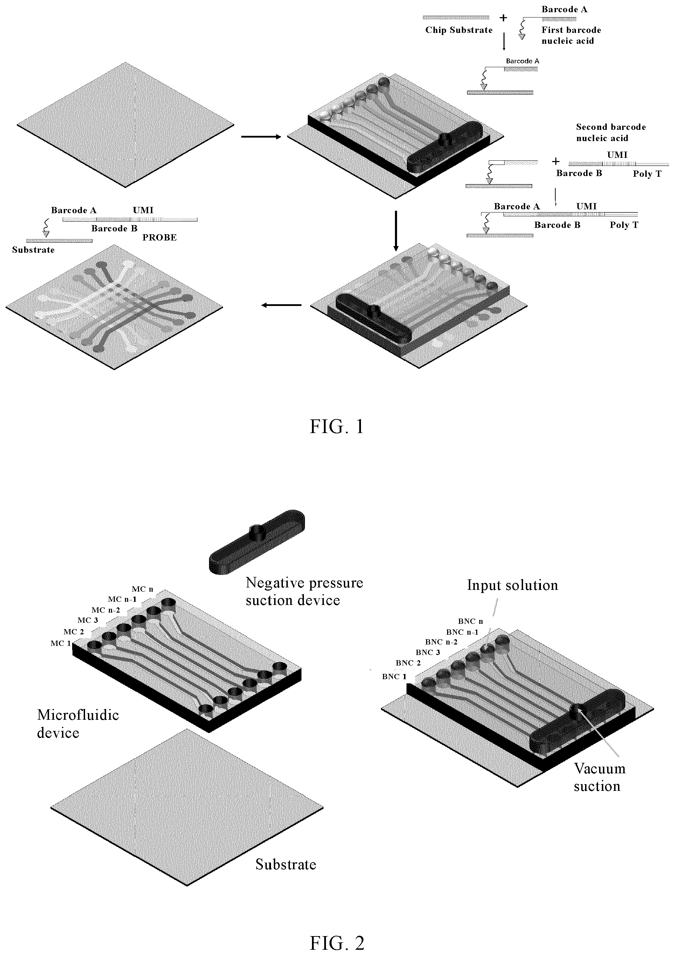

FIG. 1 shows a flow diagram of an exemplary method for preparing a biochip having an array provided by the present invention.

As shown in FIG. 1, the described method mainly comprises the following steps:
Step 1. providing a chip substrate;
Step 2. contacting and fixing (or 'immobilize' ) a first set of barcode nucleic acids (which includes a plurality of first barcode nucleic acids, wherein each first barcode nucleic acid has a different barcode sequence, i.e., Barcode A in FIG. 1) with the chip surface through a plurality of microfluidic channels set in parallel to form a plurality of first barcode strips in a first direction on the chip surface, wherein each of the first barcode nucleic acid fixed in the first barcode bands on the chip surface has a different first barcode sequence.

FIG. 2 shows an exemplary embodiment of contacting and fixing a plurality of barcode nucleic acids to a chip surface by a plurality of microfluidic channels disposed in parallel. The chip is shown at the bottom of the left panel of FIG. 2. The middle of the left panel of FIG. 2 shows a microfluidic device having a plurality of parallelly disposed microfluidic channels, i.e., microfluidic channel (MC) 1-n in FIG. 2, wherein the side of the microfluidic channel in contact with the surface of the chip, i.e., the bottom of the microfluidic channel as illustrated, allows the passage of solution and nucleic acids in solution. For example, the side of said microfluidic channel in contact with the chip surface does not have a microfluidic channel wall. The microfluidic device is placed over the surface of the chip in a first direction, and then a specified solution, such as a solution containing a barcode nucleic acid, is passed within the microfluidic channel. The upper left panel of FIG. 2 shows an exemplary device for assisting in the passage of the solution, such as a vacuum suction device utilizing negative pressure, which may be provided at the outlet of the microfluidic channel. The right panel of FIG. 2 shows the addition of barcoded nucleic acids containing different barcode sequences, i.e., barcoded nucleic acids (BNC) 1 - n in FIG. 2, through the inlet in each of the microfluidic channels. In one aspect of the present invention, the barcode sequence of said barcode nucleic acid passed through the inlet in each microfluidic channel has a known or specified nucleotide sequence.

Step 3. removing the microfluidic channel of step 2, a second set of barcode nucleic acids which comprises a plurality of second barcode nucleic acids and each of which has a different barcode sequence (Barcode B in FIG. 1), is passed through another set of a plurality of microfluidic channels disposed in parallel, in contact with a surface of the chip along a second directionthat is different from the direction of the first barcode band (typically perpendicular to the first direction), forming a plurality of second barcode bands, wherein the second barcode nucleic acid immobilized on each of the second barcode bands has a different second barcode sequence;
Under conditions that enable the first barcode nucleic acid and the second barcode nucleic acid to undergo a ligating reaction, ligating the second barcode nucleic acid to the first barcode nucleic acid on the surface of the chip where said plurality of first barcode bands produce a crossover with said plurality of second barcode bands, forming a probe.

Step 4. removing the microfluidic channel from step 3 to obtain a biochip having a probe array on the surface. Each array dot on said probe array corresponds to a location where said one of said plurality of first barcode bands forms a crossover with one of said plurality of second barcode bands. Each array dot has a probe molecule comprising a first barcode sequence and a second barcode sequence. Each array dot has a probe molecule comprising a different combination of the first barcode sequence and the second barcode sequence. In one aspect of the present invention, the first barcode sequence and the second barcode sequence of the barcoded nucleic acid passed through each of said microfluidic channels is known or specified, whereby the combination of the first barcode sequence and the second barcode sequence of the probe molecule in each array dot is also known. The spatial position of the probe molecules of each array dot in the array on the chip surface can thus be known from the first barcode sequence and the second barcode sequence of the probe molecules of the respective array dot.

In the context of the present invention, a microchip (or "microhip" or "microarray")typically refers to a solid substrate on which chemical, biological, biophysical or biochemical processes may be implemented. The chip may have microstructures or microscale structures such as channels and pore wells, electrode elements, electromagnetic elements, etc. facilitating the chemical, biological, biophysical or biochemical processes occurring on the chip. The surface of the chip may be flat or uneven. Chips with uneven surfaces may include channels or wells constructed on the surface.

The microchip may be made of any suitable material, and exemplary types of chip materials include glass, modified glass, functionalized glass, inorganic glass, microspheres (including inert and/or magnetic particles), plastics, polysaccharides, nylon, nitrocellulose, ceramics, resins, silicon dioxide, silica-based materials, carbon, optical fibers or fiber optic bundles, a wide variety of polymers in addition to the exemplary materials described above, and porous microtitre plates. plates. Specific types of exemplary plastics include acrylics, polystyrene, copolymers of styrene and other materials, polypropylene, polyethylene, polybutylene, polyurethanes, and Teflon^{™} Specific types of exemplary silica-based materials include various forms of silicon and modified silicon. Deposition of biopolymers (including nucleic acids, peptides, and/or other polymers) is typically required on the chip surface.

In the present invention, said chip surface obtained by preparation has probes (or capture probes) on the array. A probe is a single-stranded nucleotide molecule that can gene-specifically or target-specifically recognize and bind to a target nucleic acid, e.g., a nucleic acid from a tissue sample, and that has a specific nucleotide sequence, i.e., one that can selectively anneal to the targeted nucleic acid, usually a complementary nucleotide sequence. Examples of analytes in tissue samples include genomic DNA, methylated DNA, specific methylated DNA sequences, messenger RNA (mRNA), poly A mRNA, fragmented mRNA, fragmented DNA, mitochondrial DNA, viral RNA, micro RNA, in situ-synthesized PCR products, RNA/DNA heterodimers, lipids, carbohydrates, proteins, glycoproteins, lipoproteins, phosphoproteins, specific phosphorylated or acetylated variants of proteins, or viral capsid proteins. The capture probe may be a gene-specific capture probe which hybridizes, for example, to an mRNA or cDNA specifically targeted in the sample.

In the present invention, said probe has a barcode sequence for use in applications where subsequent high-throughput next-generation sequencing (NGS) or synthetic sequencing (SBS) is performed for analysis, for example in performing large-throughput sequencing analyses. In these sequences, barcode sequences are employed to mark and identify the origin of the nucleic acids of the nucleic acid sequences obtained by sequencing. Barcode molecules are used to barcode nucleic acid molecules (e.g., RNA molecules) from biological particles (e.g., cells) to generate sequencing libraries, and said sequencing libraries are subsequently sequenced to generate a plurality of sequencing read lengths. Some or all of the plurality of sequencing read lengths include barcode sequences. In these sequencing applications, cellular nucleic acids are typically amplified until the barcoded overlapping fragments in the object constitute at least 1X coverage, at least 2X, at least 3X, at least 4X, at least 5X, at least 10X, at least 20X, at least 40X, or higher coverage of a particular portion or all of the cellular genome. Once barcoded fragments are generated, they can be sequenced directly on a suitable sequencing system, such as an Illumina system. The presence of identical barcodes on multiple sequences can provide information about the origin of the sequence.

The unique barcode sequence may be relied upon in polynucleotide sequencing to identify the origin of a nucleic acid fragment and, for example, to assemble larger sequences from sequenced fragments. Barcodes may allow for identification and/or quantification of individual polynucleotide fragments during sequencing.

In the present invention, two barcode sequences are contained in a probe. The two barcode sequences can help to determine the position of the probe in the array on the surface of the chip (determining the position in the X dimension and the Y dimension, respectively), and thus also function as a position label. The barcode sequence on the probe can correspond to an array dot (feature) in the array on the chip, and can also indicate the position of a cell, including a single cell, in that tissue sample. Examples of other molecules that may be coupled to a nucleic acid tag include antibodies, antigen-binding structural domains, proteins, peptides, receptors, semi-antigens, and the like.

In the present invention, said probe further comprises one or more unique molecular identifiers (UMIs). The unique molecular identifier is a contiguous nucleic acid fragment or two or more non-contiguous nucleic acid fragments that act as a marker or identifier for a specific analyte or a capture probe that binds to a specific analyte. The UMI is a nucleic acid sequence that does not essentially hybridize with analyte nucleic acid molecules in the biological sample. The UMI may be a nucleic acid sequence comprising from about 6 to about 20 or more nucleotides within the sequence of the capture probe.

In step 2 of the method of this aspect, fix (or `immobilization') of the first set of barcoded nucleic acids to the chip may be performed using various methods known in the art. Immobilization of the nucleic acids refers to direct or indirect attachment to the chip by covalent or non-covalent bonding. In one aspect of the present invention, immobilization refers to remaining attached to the chip during reactions such as those requiring nucleic acid amplification and/or sequencing. Exemplary non-covalent linkages include, but are not limited to, non-specific interactions (e.g., hydrogen bonding, ionic bonding, van der Waals interactions, etc.) or specific interactions (e.g., affinity interactions, receptor-ligand interactions, antibody-epitope interactions, anti-biotin protein- biotin interactions, streptavidin-biotin interactions, lectin-carbohydrate interactions, etc.).

In step 3 of the methods of this aspect, the joining of the first barcoded nucleic acid with the second barcoded nucleic acid may be carried out by various methods known in the art. For example, the joining is achieved after forming a combination of three nucleic acid fragments (the first barcode nucleic acid, the second barcode nucleic acid, and the linker nucleic acid) under conditions in which a ligation reaction can be carried out, by each being complementary to the sequence of a different end of another single-stranded nucleic acid fragment (the linker nucleic acid).

In one aspect of the present invention, said first barcode nucleic acid comprises a first barcode fragment. In one aspect of the present invention, said first barcode nucleic acid has at the 5' end a primer fragment for a subsequent amplification reaction, for example a universal primer sequence for use in a known sequencing method. In one aspect of the invention, said first barcode nucleic acid has at the 5' end a motif or sequence for attachment to a chip surface. For example, where the chip surface is modified with aldehyde, the first barcode nucleic acid has an amino group at the 5' end. In one aspect of the invention, said first barcode nucleic acid has a ligand that can form a specific interaction with a factor modified on the chip, for example said factor and ligand are antibody-epitope, anti-biotin protein-biotin, streptavidin-biotin, lectin-carbohydrate, respectively.

In one aspect of the present invention, said second barcode nucleic acid comprises a second barcode fragment. In one aspect of the present invention, said second barcode nucleic acid has a capture fragment at the 3' end for recognizing and binding to a target in a biological sample, such as a fragment for recognizing and binding to mRNA or cDNA, such as a poly-T sequence for recognizing mRNA.

In one of the aspects of the present invention, the 3' end of said first barcode nucleic acid has a first linker fragment (3' linker fragment) for linking to a second barcode nucleic acid. In another one of these aspects of the present invention, said 5' end of said second barcode nucleic acid has a second linkage fragment (5' linkage fragment) for linkage to the first barcode nucleic acid. In yet another one of these aspects of the present invention, said first linker fragment and second linker fragment each form a complement to one end of the linker nucleic acid, and under conditions facilitating ligation reaction (e.g., in the presence of a T4 ligase, etc.), the first linker fragment and the second linker fragment form a combination with the linker nucleic acid to achieve the ligation of the first barcode nucleic acid and the second barcode nucleic acid.

The microchips prepared by the method provided by the present invention can be used for the analysis of molecules contained in cells, including nucleic acids and proteins, on tissue samples, in particular thin sections of tissues, e.g. by PCR, mass spectrometry, next-generation sequencing, or ELISA, to obtain their expression and spatial information.

"Sequencing" generally refers to methods and techniques for determining the sequence of nucleotide bases in one or more polynucleotides. A polynucleotide may be, for example, a nucleic acid molecule such as deoxyribonucleic acid (DNA) or ribonucleic acid (RNA), including variants or derivatives thereof (e.g., single-stranded DNA). Sequencing may be performed by various systems currently available, such as, but not limited to, sequencing systems through Illumina, Pacific Biosciences, Oxford Nanopore, or Life Technologies. Alternatively or additionally, sequencing may be performed using nucleic acid amplification, polymerase chain reaction (PCR) (e.g., digital PCR, quantitative PCR, or real-time PCR), or isothermal amplification. Such systems may provide a plurality of raw genetic data corresponding to genetic information of a subject (e.g., a human), such as samples provided from the subject generated by the system. In some examples, such systems provide a sequencing read length (also referred to herein as a "read length"). The read length may include a string of nucleic acid bases that correspond to the sequence of the sequenced nucleic acid molecule. In some embodiments, the systems and methods provided herein may be used in conjunction with proteomic information.

The microchips prepared in the present invention are suitable for use in analyzing nucleic acid information of cells of biological samples, and in particular spatial transcriptomics information of biological tissue samples. A "tissue sample" suitable for the present invention includes tissue obtained from a subject, fixed, sectioned and mounted on a flat surface. The tissue sample may be, for example, a formalin-fixed paraffin-embedded (FFPE) tissue sample or a fresh tissue sample or a frozen tissue sample. The methods disclosed herein may be performed before or after staining the tissue sample. For example, after hematoxylin and eosin staining, the tissue sample may be spatially analyzed according to the methods provided herein. The methods can include analyzing the histology of the sample (e.g., using hematoxylin and eosin staining) and then spatially analyzing the tissue. Tissue sections fixed in formalin and embedded in paraffin (FFPE) typically include fixing tissue obtained from a subject in formaldehyde (e.g., 3%-5% formaldehyde in phosphate-buffered saline) or Bouin solution, embedding in wax, cutting into thin sections, and then mounting on a flat surface, such as a microscope slide for biopsy.

The use of the microchips of the present invention, comprise contacting a tissue section with the chips on which probes recognize and bind nucleic acids, particularly mRNA, from cells in the tissue. Subsequent analyses include reverse transcription and amplification, among others, and may be performed by high-throughput next-generation sequencing (NGS) or synthetic sequencing (SBS).

In some embodiments, nucleic acids from tissue sections (e.g., formalin-fixed paraffin-embedded (FFPE) tissue sections) are transferred to the array and captured onto the array by hybridization to a capture probe. In some embodiments, the capture probe may be a universal capture probe that hybridizes, for example, to an articulator region in a nucleic acid sequencing library, or to a poly-A tail of mRNA. In some embodiments, the capture probe may be a gene-specific capture probe that hybridizes, for example, to an mRNA or cDNA specifically targeted in the sample.

In some embodiments, nucleic acids from tissue sections (e.g., FFPE sections) are transferred to the array and captured on the array by single-stranded ligation to a universal articulator oligonucleotide. In other embodiments, nucleic acids on a chip may be transferred to a tissue section (e.g., an FFPE section). Methods known in the art may be used to enable probes bound to the chip to enter cells on the tissue with which they are in contact after being dislodged in solution. For example, a photolyzable connector or the like may be added to the binding of the nucleic acid probe to the chip, or the nucleic acid probe may be bound to the chip by a pH-sensitive connector, and then the nucleic acid probe may be detached from the chip by changing the pH of the solution.

With the chip of the present invention and the use thereof, the spatial position of the probe molecule in the array on the surface of the chip can be known from the first barcode sequence and the second barcode sequence of the probe molecule that each array dot has, and thereby information on the position in said tissue of the cell in which the nucleic acid molecule is contained can be obtained.

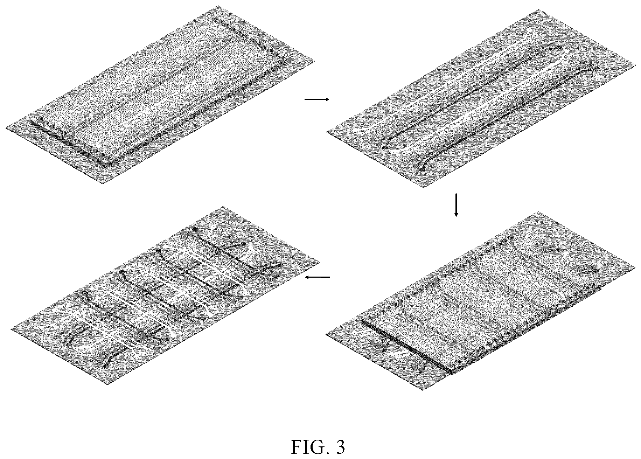

The method for preparing a chip having a probe array provided by the present invention enables parallel synthesis of a plurality of chips having arrays of identical coding regions on the same substrate. As shown in FIG. 3, a plurality of sets of identical first bar code bands and second bar code bands can be formed in a first direction and a second direction of the chip substrate as desired, thereby obtaining a plurality of chips having probes defined by identical first bar code and second bar code sequences at corresponding array points.

In one of the embodiments of the present invention, the inventor unexpectedly found that, in step 2 of the aforementioned method (i.e., in step A of a method provided herein for preparing a biochip having arrays), i.e., when fixing the nucleic acid of said first barcode nucleic acids on the surface of the chip, the efficiency of fixing of the nucleic acids of the first set of barcode nucleic acids onto the chip can be significantly improved by linking the nucleic acid of said first set of barcode nucleic acids with nucleic acids (referred to herein as chip surface linker nucleic acids or chip surface linkers) that have been fixed on the surface of the chip (the entire surface of the chip or sections of the surface of the chip having the same position as the first set of barcode nucleic acids) through a nucleic acid-to-nucleic acid linkage reaction (e.g., with a nucleic acid ligase and a nucleic acid linker fragments bridging the two nucleic acids to be linked), which resulting in a significant increase in the density of probes at the array's dots and a significant improvement in uniformity of the probes at the array's dots. Without being limited by this theory, the inventors believe that this is due to the greater efficiency and stability of the nucleic acid-to-nucleic acid linkage reaction than other ways of fixing nucleic acids to the chip surface (e.g., covalent bonding ways, etc.), and/or due to being able to reduce the length of each nucleic acid fragment on the probes, and to increase the efficiency of linkage between each of the individual fragments during the preparing. Accordingly, in one embodiment of the present invention, step A is preceded by a pre-step comprising pre-immobilizing on the surface of the chip (e.g., on the entire surface of the chip) a chip-surface linker nucleic acid for the use of joining with the first set of barcode nucleic acids. The chip surface linker nucleic acid may be immobilized on the chip surface by chemical bonding means. The chemical bonding means includes any chemical group reactions, for example, amino-aldehyde group reaction and the like, or covalent cross-linking. The surface of the chip may be encapsulated by a surface chemical reaction with an amino group, an aldehyde group, an epoxy group, an isothiocyanate group, a sulfhydryl group, a silane and other reactive groups and the like. The end (usually the 5' end) of said chip surface linker nucleic acid connected to the chip surface then has a group that forms a chemical bond with the encapsulated reactive group. In yet another one of the embodiments of the present invention, the 3' end of said chip surface linker nucleic acid has a linker fragment for the use of linking with the first barcode nucleic acid via a single-stranded linker nucleic acid. In yet another one of the embodiments of the present invention, wherein the 5' end of said first barcode nucleic acid has a linkage fragment for linking to said chip surface linker nucleic acid via a single-stranded linker nucleic acid, the 3' end of said chip surface linker nucleic acid has a linkage fragment for linking to said first barcode nucleic acid via said single-stranded linker nucleic acid, said linkage fragment at the 3' end of said chip surface linker nucleic acid and said linkage fragment at the 5' end of said first barcode nucleic acid being reverse complementary to the sequences of the ends of said single-stranded linker nucleic acid, respectively. And, said 3' end of said first barcode nucleic acid has a linkage fragment for linkage to a second barcode nucleic acid via a single-stranded linkage nucleic acid, said 5' end of said second barcode nucleic acid has a linkage fragment for linkage to a first barcode nucleic acid via said single-stranded linkage nucleic acid, said 3' end of said first barcode nucleic acid ' end of said first barcode nucleic acid and the linkage fragment at the 5' end of said second barcode nucleic acid are each reverse complementary to the sequences of said single-stranded linker nucleic acid at each end of said single-stranded linker nucleic acid. In yet another one of the embodiments of the present invention, said chip surface linker nucleic acid has at the 5' end a primer fragment for subsequent amplification reactions, for example a universal primer sequence for use in known sequencing methods.

The present invention also provides a biochip for analyzing nucleic acid information of biological samples. In one aspect of the present invention, said biochip for analyzing nucleic acid information of a biological sample is prepared by the aforementioned method. In one aspect of the present invention, said chip for analyzing nucleic acid information of a biological sample has a surface having probes forming an array, said array comprising orthogonal rows and columns of probes, said probes in said array each having a different sequence that can be used to indicate the spatial location of said probes. In one aspect of the present invention, said probes comprise a first barcode and a second barcode. In yet another one of these aspects of the present invention, each row of probes of said array of probes has the same first barcode and each column of probes has the same second barcode; each row of probes has a different first barcode and each column of probes has a different second barcode from the other row or column. In one aspect of the present invention, said chip for analyzing nucleic acid information of a biological sample has chip surface linker nucleic acids on its entire surface. In yet another one of these aspects of the present invention, the 5' end of each probe in said probe array is said chip surface linker nucleic acid. In yet another one of these aspects of the present invention, each probe in said probe array comprises, from the 5' end to the 3' end, said chip surface linker nucleic acid, a first barcode, a second barcode, and a capture fragment for recognizing and binding to a target nucleic acid in a biological sample. In yet another one of these aspects of the present invention, each probe in said probe array includes a primer fragment at the 5' end for an amplification reaction. In yet another one of these aspects of the present invention, the sequence of each probe in said probe array further comprises a unique molecular identifier (UMI).

The present invention also provides methods for analyzing spatial transcriptomics information of a biological tissue sample, said methods comprising contacting an array of the aforementioned chip with a tissue sample. A "tissue sample" for the purposes of the present invention includes tissue obtained from a subject, fixed, sectioned and mounted on a flat surface. The tissue sample may be, for example, a formalin-fixed paraffin-embedded (FFPE) tissue sample or a fresh tissue sample or a frozen tissue sample. The methods of the present invention may be performed before or after staining the tissue samples. For example, after hematoxylin and eosin staining, the tissue sample may be used for spatial transcriptomics analyzed according to the methods provided herein. The methods may include analyzing the histology of the sample (e.g., using hematoxylin and eosin staining) and then spatially analyzing the tissue. Tissue sections fixed in formalin and embedded in paraffin (FFPE) typically include fixing tissue obtained from a subject in formaldehyde (e.g., 3%-5% formaldehyde in phosphate-buffered saline) or Bouin solution, embedding it in wax, cutting it into thin sections, and then mounting it on a flat surface, such as a microscope slide for biopsy. The method of the present invention involves contacting the tissue section with a probe array on a microchip, which recognizes and binds nucleic acids, particularly mRNA, from the cells in the tissue. Subsequent analyses include reverse transcription and amplification, among others, and can be performed by high-throughput next-generation sequencing (NGS) or synthetic sequencing (SBS).

### Example 2 Preparation of the biochip (sectional surface modification)

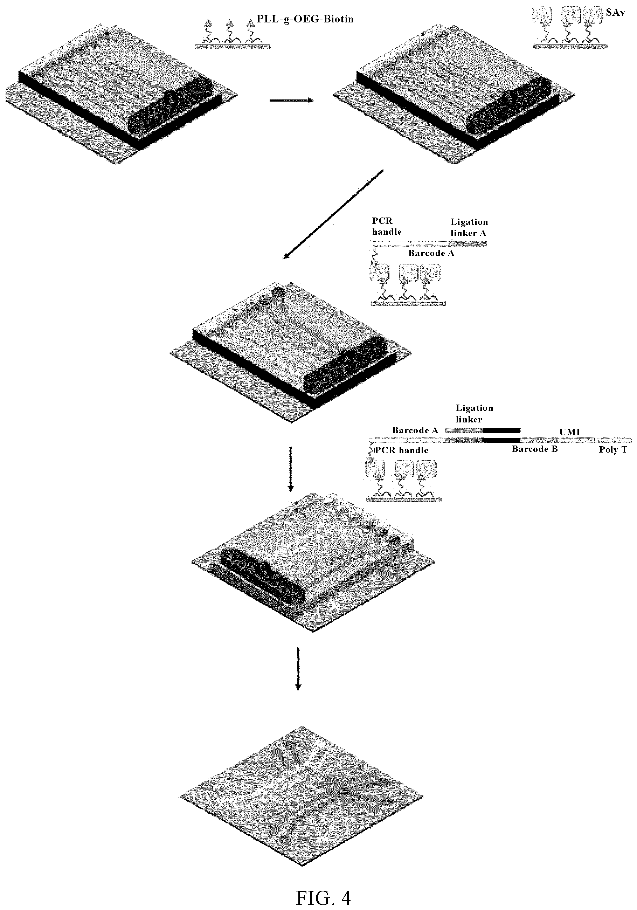

FIG. 4 shows a flowchart of an exemplary embodiment of a method for preparing a biochip provided by the present invention.

In this example, a glass slide is used as the chip substrate.

A device including a plurality of microfluidic channels disposed in parallel with openings at the bottom of the microfluidic channels is prepared by a soft lithography process made of polydimethylsiloxane (PDMS) as shown in FIG. 2.

The device of the microfluidic channels includes about 50-500 parallel set microfluidic channels. The width of each of said parallel provided microfluidic channels is about 2-200 µm, preferably about 5-50 µm, most preferably about 5-25 µm, for example, about 5 µm, 10 µm, or 50 µm.the spacing between each of the adjacent microfluidic channels is about 5-400 µm, preferably about 10-100 µm, most preferably about 10-50 µm, for example, about 20 µm, 50 µ m or 100 µm.

Synthesize PLL-PEG-biotin and then prepare a 1 mg/ml solution of PLL-PEG-biotin in PBS.

Synthesize 100 5' end biotin-modified Set I barcoded nucleic acids with the following sequences: 
5' biotin- CTACACGACGCTCTTCCGATCT 12345678
CTCTTTCCCTACACGACGCTCTT- 3'
 wherein "12345678" denotes a barcode fragment having 8 nucleotides, wherein the sequence of said 8 nucleotides is known (specified), and wherein the sequences of the first barcode of each of said 100 Set I barcode nucleic acids are different from each other. The CTACACGACGCTCTTCCGATCT on the 5' side of this barcode fragment is the primer fragment used for amplification reactions in subsequent sequencing procedures. The CTCTTTCCCTACACGACGCTCTT on the 3' side of this barcode fragment is a linker fragment used to form a linkage with the second barcode fragment.

Synthesize 100 second set of barcode nucleic acids having the following sequences: 
5’ phosphorylated -GAGTGATTGCTTGTGACGCCTT 87654321 NNNNNNNNNN
TTTTTTTTTTTTTTTTTTTTTTTTTTTTTTVN- 3'
 wherein "87654321" denotes a barcode fragment having 8 nucleotides, wherein the sequence of said 8 nucleotides is known (specified) and the sequences of the second barcode of said 100 second set of barcoded nucleic acids are different from each other. In one aspect of the present invention. The barcode fragments of each of the barcode nucleic acids of the second set of barcode nucleic acids are identical to the barcode fragments of each of the barcode nucleic acids of the first set of barcode nucleic acids. The GAGTGATTGCTTGTGACGCCTT on the 5' side of this barcode fragment is a linker fragment used to form a linkage with the first barcode fragment. The 3' side of this barcode fragment has a polyT VN sequence that can be used to bind mRNA.

The nucleic acid linker for use to link the first barcode nucleic acid to the second barcode nucleic acid was synthesized with the following sequence:
5'-AAGGCGTCACAAGCAATCACTCAAGAGCGTCGTGTAGGGAAAGAG- 3'

The PDMS microfluidic device is attached to the slide to provide a flow channel. Depending on the interface characteristics, a clamping tool can be used to press the microfluidic device to the substrate slide to improve the sealling. One end of the microfluidic channel is the solution inlet and the other end is connected to the vacuum suction device through an interface.

The first PDMS microfluidic device was fit to the slide, passed through ethanol to remove air bubbles, and then rinsed once by using the PBS buffer, and then 1 mg/ml of PLL-PEG-biotin solution was added to the flow channel and the reaction was carried out for about 30 min at room temperature. A low negative pressure was maintained to keep the fluid at a low speed, allowing the consumed reactants to be replenished.

Upon completion of the reaction, PBS buffer was passed to clean the flow channel, followed by streptavidin, which filled the flow channel and was left to reaction for 10 minutes at room temperature. Upon completion of the reaction, sectional modification of the chip at the sections of the chip surface which were covered by the channels was achieved.

PBS buffer was passed for cleaning. After washing, biotinylated Set I barcoded nucleic acids are passed within the microfluidic channel: 15 µM of one of the first barcoded nucleic acids is passed into each flow channel (the first barcoded nucleic acid in each flow channel has a different barcode sequence than the first barcoded nucleic acids in the other flow channels). Fill the flow channels and leave for 10 minutes at room temperature. Upon completion of the reaction, fixing of the first set of barcoded nucleic acids on the chip surface which were covered by the channels was achieved, forming the first set of barcode strips.

The flow channel was rinsed with PBS buffer and ultrapure water, and after rinsing, the microfluidic device was removed and the chip was air-dried at room temperature.

After drying, another PDMS microfluidic device was fit to the slide along a direction that formed an orthogonal direction to the flow channel of the first PDMS microfluidic device. After passing buffer into the flow channel to expel gas from the flow channel, 15 µM of the second set of barcode nucleic acids was passed: one second barcode nucleic acid (the second barcode nucleic acid of each flow channel has a different barcode sequence than the second barcode nucleic acid of the other flow channels) and the nucleic acid linker and T4 ligase were passed into each flow channel. The flow channels were filled and left for reaction for 30 minutes at room temperature.

After completing the ligation reaction, buffer was passed through and the channels were cleaned with ultrapure water. After that, the device was removed and the substrate was dried. The chip preparation was completed.

### Example 3 Preparation of the biochip (whole surface modification)

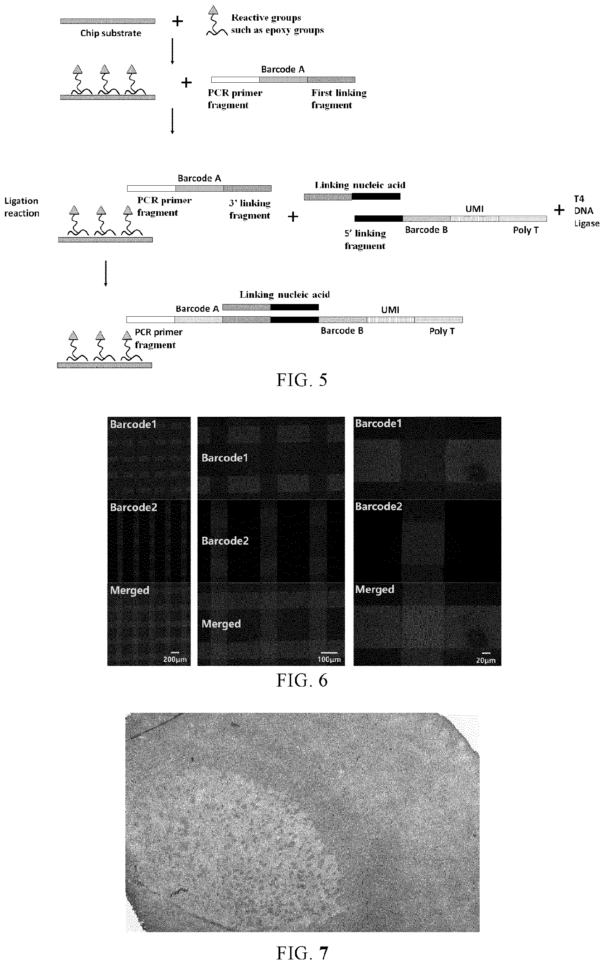

FIG. 5 shows a flow chart of another exemplary embodiment of a method for preparing a biochip provided by the present invention.

In this embodiment, a glass slide is used as the chip substrate, and the surface of the chip is modified with reactive groups such as amino groups, aldehyde groups, epoxy groups, isothiocyanate groups, sulfhydryl groups, silanes, and other reactive groups by a surface chemical reaction.

In this Example, a commercially-available glass sheet modified with epoxy groups (Nexterion^{®} Slide E) is used as the chip substrate.

One hundred 5' end amino modified Set I barcoded nucleic acids with the following sequences were synthesized: 
5'	amino-CTACACGACGCTCTTCCGATCT	12345678
CTCTTTCCCTACACGACGCTCTT- 3'
 wherein "12345678" denotes a barcode fragment having 8 nucleotides, wherein the sequence of said 8 nucleotides is known (specified). The sequence of said barcode fragment (referred to as the first barcode) of each of said 100 first barcode nucleic acids are different from each other, and the sequence of the first barcode of each first group barcode nucleic acid is known (specified), wherein the underlined T base is FITC modified. In this embodiment, fluorescence modification of the barcode fragments and detection of the resulting fluorescence signal is used for observation or production quality control of each step of incorporation of the barcode fragments in the chip synthesis. In other embodiments, fluorescence modification of the barcode fragment may not be present.

Synthesize 100 Set II of barcode nucleic acids having the following sequences: 
5’ phosphorylated -GAGTGATTGCTTGTGACGCCTT 87654321 NNNNNNNNNN
TTTTTTTTTTTTTTTTTTTTTTTTTTTTTTVN- 3'
 wherein "87654321" denotes a barcode fragment having 8 nucleotides, wherein the sequence of said 8 nucleotides is known (specified), wherein the sequences of the barcode fragments (referred to as second barcodes) of said 100 Set II barcode nucleic acids are different from each other, and wherein the sequence of the second barcodes of each Set II barcode nucleic acid is known (specified). The underlined T base is Cy3 modified.

Synthesize 1 nucleic acid linker with the following sequence:
5'-CAAGCAATCACTCGAAGAGCGTCGTGT- 3'

The PDMS microfluidic device described in Example 2 was fixed to the slide to provide a flow channel. A clamping tool can be used to press the microfluidic device to the substrate slide to improve the sealling. One end of the microfluidic channel is the solution inlet and the other end is connected to the vacuum suction device through an interface.

The first PDMS microfluidic device was fitted to the slide, passed through ethanol to remove air bubbles, and rinsed once more with PBS buffer, and then 15 µM of the first set of barcode nucleic acids (dissolved in 300 mM sodium phosphate buffer, pH 8.5) was added to the flow channel: one type of the first barcode nucleic acid was passed through each flow channel (the first barcode nucleic acid of each flow channel has a different barcode sequence from the first barcode nucleic acid of the other flow channels). After filling the flow channels, the slides were placed in a saturated sodium chloride wet box at 35°C and left to stand for three hours at room temperature. After the reaction was completed, the flow channel was rinsed with 1× PBS buffer for 1 minute. The PDMS microfluidic device was then removed and the modified slides were sequentially washed using 0.1 % Triton X-100, 1 mM HCl, 100 mM KC1, and then closed using 0.1 M Tris pH 9.0, 50 mM ethanolamine, and 0.1% SDS at 50°C. The substrate was rinsed with deionized water for 1 min after the end of containment, and then the substrate was blown dry with nitrogen. A fluorescence microscope was used to observe the FITC fluorescence signal of the first barcode nucleic acids to determine the completion of the reaction and to achieve the fixation of the first set of barcode nucleic acids on the chip surface which were covered by the channels was achieved to form the first set of barcode bands.

Another PDMS microfluidic device is fixed to the slide along a direction that forms an orthogonal direction to the flow channel of the first PDMS microfluidic device. After passing buffer into the flow channel to expel gas from the flow channel, 15 µM of the second set of barcode nucleic acids was passed: one second barcode nucleic acid (the second barcode nucleic acid of each flow channel has a different barcode sequence from the second barcode nucleic acid of the other flow channels) and nucleic acid linker and T4 ligase were passed into each flow channel. The flow channels were filled and left to stand at 37°C for 30 min.

After completion of the ligation reaction, 1× PBS buffer was passed through and the runners were cleaned with ultrapure water. After that, the device was removed and the substrate was rinsed with deionized water for 1 min, and then the substrate was blown dry with nitrogen. A fluorescence microscope was used to observe the Cy3 fluorescence signal of the second barcode nucleic acid to determine the completion of the reaction and the effect of linkage reaction between the second barcode nucleic acid and the first set of barcode nucleic acids at the intersection of the flow channel to form the barcode array, and thus complete the chip preparation.

The efficiency of the ligation reaction between each barcode nucleic acid as well as the intensity (density) and uniformity of the probes of the prepared chip were evaluated by observing the fluorescence signals on the prepared chip. FIG. 6 shows a plot of the observation of each barcode nucleic acid ligation reaction under a microscope (Olympus bx53). As shown in FIG. 6, the FITC-modified fluorescent signals indicating the first set of barcoded nucleic acids are shown on the horizontal array of the chip; the Cy3-modified fluorescent signals indicating the second set of barcoded nucleic acids are shown on the vertical array of the chip.

### Example 4 Tissue sample preparation and staining

### (I) Tissue OCT embedding

Collect fresh mouse brain tissue samples, rinse the tissue surface residue with pre-cooled PBS solution or saline, and then absorb the liquid with clean absorbent paper. Place the tissue in the embedding tank and add OCT embedding agent until the tissue is completely covered. Make sure there are no air bubbles around the tissue and place the embedding tank on dry ice until the OCT is completely frozen.

### (ii) Frozen Sections

Set the temperature of the frozen sectioning machine to -20°C for the chamber and -10° C for the sample head. Before sectioning, the frozen tissues and substrates were first equilibrated in the -20°C freezer sectioning machine chamber for more than 30 minutes, and then frozen sectioning was performed in the freezer sectioning machine chamber with a thickness of 10 µm.

### (iii) Tissue fixation and HE staining

Attach the cut tissue sections to the barcode array-modified substrate prepared in Example 3, and then incubate at 37°C for 1 minute. Completely immerse the adhered tissue substrate in pre-cooled methanol and fix it at -20°C for 30 minutes. At the end of fixation, the substrate was removed, the liquid on the backside was wiped dry, and 500 µl of isopropanol was added dropwise to the tissue sections and incubated for 1 min at room temperature. After 1 min, the isopropanol was removed and then air-dried for 5-10 min at room temperature.

Add 1 ml of hematoxylin to evenly cover the tissue sections on the substrate and incubate for 7 minutes at room temperature. Remove hematoxylin reagent, wash the substrate by immersing it in RNase-free Water and air dry. Add 1 ml of bluing solution and incubate for 2 minutes at room temperature. Remove the bluing solution, wash the substrate by immersing it in RNase-free Water and dry the liquid on the back of the substrate. Add 1 ml of Eosin mixture and incubate for 1 minute at room temperature.

Remove the eosin, wash the substrate by immersing it in RNase-free Water and air dry until the tissue is opaque. After incubating the slides for 5 min at 37°C, bright field imaging was performed.

FIG. 7 shows the tissue images after HE staining.

### (iv) Tissue permeabilization

Assemble a fixture chamber onto the tissue chip, making sure that the tissue sections are located inside the corresponding chambers. Add 70ul of permeabilizing enzyme (0.1% pepsin diluted in 0.1N HCl) to the chamber to permeabilize the tissue at 37°C, remove the permeabilizing enzyme and then wash with 0.1× SSC.

### Example 5 Reverse transcription reaction, library construction and sequencing of tissue sample sections

Add 70 µl of reverse transcription mix to the chamber in Example 4. The reverse transcription mix comprises: 1x first strand buffer, 5 mM DTT, 500 µM dNTP, 0 .19 µg/µl BSA, 1% DMSO, 2.5 µM Template Switch Oligo, 20 U/ µl Superscript III, and 2 U/ µl RNase inhibitor.

The Template Switch Oligo sequence was:
5'Biotin-AAGCAGTGGTATCAACGCAGAGTACATrGrGrG-3'

In the Template Switch Oligo, the penultimate 1, 2 and 3 bases were modified with riboguanine nucleosides.

After sealing the chamber with adhesive tape, the reaction mixture was placed on a temperature-controlled plate and set to approximately 50°C for reverse transcription for 16 hours.

At the end of reverse transcription, aspirate the reverse transcription mixture from the chamber. The chamber was incubated for 5 min at room temperature with 70 µl of 0.08 M KOH, and then washed once with 100 ul of RNase-free water.

Add cDNA second strand synthesis reaction solution to the cleaned chamber. The second strand synthesis reaction solution included 1×Kapa HiFi Hotstart ReadyMix, 0.8µM Second Strand Primer. After sealing the chamber with tape, the chamber was placed on a temperature-controlled plate and set to about 37°C for cDNA second strand synthesis for 30 min.

The Second Strand Primer sequence is:
5'-AAGCAGTGGTATCAACGCAGAGTACAT-3'

At the end of the reaction, the second strand synthesis reaction mixture inside the chamber was aspirated and then washed once by adding 100ul of RNase-free Water. Next, 70 µl of 0.08 M KOH was added to the chamber and incubated for 10 min at room temperature. Prepare several new 1.5 ml centrifuge tubes by adding 10 µl Tris (1 M, pH 7.0). Transfer 70 µl of sample from the chamber to the corresponding Tris-containing centrifuge tubes and mix well to complete the preparation of second-strand of cDNA.

### Amplification of cDNA

A new 1.5 ml centrifuge tube was placed on ice to prepare the PCR amplification reaction solution. The PCR reaction solution consisted of 1 ×Kapa HiFi Hotstart ReadyMix, 0.8 µM cDNA Forward Primer, 0.8 µM cDNA Reverse Primer, and 35 µl cDNA template, in a total volume of 100 µl. cDNA amplification was carried out by the following protocol:

| | | |
|---|---|---|
| 98 °C | 3min | 1 |
| 98 °C | 15s | **15 circles** |
| 63 °C | 20s | |
| 72 °C | 1min | |

Where the cDNA Forward Primer sequence was:
   5'-CTACACGACGCTCTCTTCCGATC - 3'
The cDNA Reverse Primer sequence is:
   5'-AAGCAGTGGTATCAACGCAGAG - 3'

The results showed a CT value of about 10.6.

After amplification, the amplified product was purified using AMpure SPRIselect Beads. The amplified product was subjected to library building/sequencing.

The foregoing is only a preferred embodiment of the present invention and is not intended to limit the present invention, and any modifications, equivalent substitutions, improvements, etc., within the spirit and principles of the present invention shall be included in the scope of protection of the present invention.

## Claims

1. A method for preparing a biochip having an array, comprising steps of:
A. fixing a first set of barcode nucleic acids on the surface of a chip through a plurality of microfluidic channels set in parallel to form a plurality of first barcode strips in a first direction, wherein said first set of barcode nucleic acids comprise a plurality of first barcode nucleic acids having different barcode sequences, each said first barcode strip contains one kind of first barcode nucleic acid, and each said kind of first barcode nucleic acid fixed in each first barcode strip has a different barcode sequence;
B. applying a second set of barcode nucleic acids in a second direction to said plurality of first barcode strips in the first direction on the chip surface through a plurality of microfluidic channels set in parallel to form a plurality of second barcode strips, said second set of barcode nucleic acids comprise a plurality of second barcode nucleic acids having different barcode sequences, each said second barcode strip contains one kind of second barcode nucleic acid, and each said kind of second barcode nucleic acid contained in each second barcode strip has a different barcode sequence;
C. under conditions that cause the first barcode nucleic acid and the second barcode nucleic acid to undergo a joining reaction, joining the second barcode nucleic acid to the first barcode nucleic acid on the surface of the chip where said plurality of first barcode strips intersect said plurality of second barcode strips to generate probes, said probes forming arrays of dots, each dot containing a probe with a sequence different from each other.

2. The method of claim 1, wherein said first set of barcode nucleic acids or second set of barcode nucleic acids are delivered and fixed to the chip surface using a microfluidic device having a plurality of microfluidic channels set in parallel, wherein the side of said microfluidic channel in contact with the chip surface is set to allow passage of solution or nucleic acids in solution, wherein one of the first set of barcode nucleic acids or the second set of barcode nucleic acids containing different barcode sequences is added into each microfluidic channel of said microfluidic device.

3. The method of claim 1, wherein said first barcode nucleic acid of said first set of barcode nucleic acids comprises a first barcode fragment, and comprises a primer fragment at the 5' end for use in an amplification reaction.

4. The method of claim 3, wherein said first barcode nucleic acid of said first set of barcode nucleic acids has a moiety at the 5' end for attachment to the surface of the chip.

5. The method of claim 1, wherein said second barcode nucleic acid in said second set of barcode nucleic acids comprises a probe fragment at the 3' end for recognizing and binding a target nucleic acid in the biological sample (e.g., a fragment for recognizing and binding mRNA or cDNA, e.g., a poly-T sequence) and a second barcode fragment.

6. The method of claim 5, wherein said second barcoded nucleic acid in said second set of barcoded nucleic acids further has a unique molecular identifier (UMI).

7. The method of claim 1, wherein said first barcode nucleic acid has a first linking fragment at the 3' end for linking to the second barcode nucleic acid via a single-stranded linking nucleic acid, and said second barcode nucleic acid has a second linking fragment at the 5' end for linking to the first barcode nucleic acid via said single-stranded linking nucleic acid, said first linking fragment and second linking fragment are each complementary to a sequence at each end of said single-stranded linking nucleic acid.

8. The method of claim 1, wherein the probe formed in step C comprises a capture fragment at the 3' end for identifying and binding a target nucleic acid in the biological sample, and a first barcode fragment and a second barcode fragment, preferably said probe further having a primer fragment at the 5' end for use in an amplification reaction.

9. The method of claim 1, wherein the sequence of the barcode fragment of each of said first barcode nucleic acids of said first set of barcode nucleic acids and the sequence of the barcode fragment of each of said second barcode nucleic acids of said second set of barcode nucleic acids is designated.

10. The method of claim 1, wherein the concentration of nucleic acid in the flow channel in step A or B is about 0.1-100 µM, for example, about 1-20 µM.

11. The method of claim 1, wherein in step A, said nucleic acid of said first set of barcoded nucleic acids is fixed on the chip surface by chemical bonding means, said chemical bonding means being any chemical group reactions, for example, amino-aldehyde group reaction and the like, or covalent cross-linking.

12. The method of claim 1, wherein the width of each channel of said microfluidic channels set in parallel in step A and step B is about 2-200 µm, preferably about 5-50 µm, most preferably about 5-25 µm, for example about 5 µm, 10 µm or 50 µm,
and,
spacing between each adjacent microfluidic channel of the microfluidic channels set in parallel is about 5-400 µm, preferably about 10-100 µm, most preferably about 10-50 µm, such as about 20 µm, 50 µm or 100 µm.

13. A method for analyzing spatial transcriptomic information of a biological tissue sample by using a biochip having an array prepared by the method of any one of claims 1-12, said method comprising: contacting the array of the biochip with the tissue sample, the probes on the array recognizing and binding nucleic acids, in particular mRNA, of cells in the tissue, followed by reactions such as reverse transcription and amplification, optionally by high throughput next generation sequencing (NGS) or synthetic sequencing (SBS) to further analyze the obtained nucleic acids.
